# EUROPÄISCHE PATENTANMELDUNG

(11) **EP 0 651 994 A1**
(43) Veröffentlichungstag der Anmeldung: **10.05.1995**
(21) Anmeldenummer: 94117607.5
(22) Anmeldetag: 08.11.1994
(51) Int. Cl.: A61K 9/107

(54) **Flüssige orale Zubereitungen von fettlösliche Arzneimittel**

(30) Priorität: 08.11.1993 DE 4338046
(71) Anmelder: Dietl, Hans, Dr., D-8202 Bad Aibling (DE)
(72) Erfinder: Dietl, Hans, Dr., D-8202 Bad Aibling (DE)
(74) Vertreter: Reinhard, Horst, Dr.

(57) **Zusammenfassung**

Es wird eine flüssige pharmazeutische Zubereitung zur oralen Applikation offenbart, die ein oder mehrere fettlösliche Arzneimittel, ein oder mehrere Öle natürlicher Herkunft, Phosphatidylcholin und/oder Phosphatidylethanolamin und Wasser enthält.

## Beschreibung

Die Erfindung betrifft eine neue, fettlösliche Arzneimittel enthaltende flüssige pharmazeutische Zubereitung zur oralen Applikation, ein Herstellungsverfahren für diese pharmazeutische Zubereitung und ihre Verwendung zur oralen Applikation.

Es ist seit langem bekannt, Arzneimittel zur oralen Anwendung in Form von Tabletten, Kapseln oder Dragees mit einer verzögerten Freigabe des Wirkstoffs im Magen-Darm-Trakt einzusetzen. Dazu werden beispielsweise magensaftresistente Tabletten, Kapseln oder Dragees hergestellt, die mit Überzugsmaterialien versehen sind, welche dem Angriff des Magensafts widerstehen, sich jedoch im Dünndarm lösen und damit das Arzneimittel erst im Dünndarm freisetzen. Dadurch erhält man magensaftresistente, dünndarmlösliche Arzneiformen. Diese werden vor allem dann eingesetzt, falls sich der verwendete arzneiliche Wirkstoff im sauren Milieu des Magens zersetzen kann und damit vor dem Angriff des Magensafts geschützt werden muß. Ein weiterer Grund für den Einsatz von magensaftresistenten Arzneiformen ist es, Reizungen des Magens durch das Arzneimittel zu vermeiden. Häufig verwendete Überzugsmaterialien zur Herstellung magensaftresistenter, aber dünndarmlöslicher Arzneiformen sind beispielsweise Schellack, Celluloseacetaphtalat sowie kationische Polymerisate aus Dimethylaminoäthylmethacrylat und anderen Methacrylsäureestern.

Neben magensaftresistenten, dünndarmlöslichen festen Arzneiformen (z. B. Tabletten, Dragees, Kapseln) stehen retardierte Arzneiformen zur Verfügung, bei denen der arzneiliche Wirkstoff in verzögerter Weise freigesetzt wird. Damit wird ein langsamerer Anstieg des Wirkstoffs im Blut sowie eine länger anhaltende Wirkungsdauer erreicht.

Neben den offensichtlichen Vorteilen der hier beschriebenen festen Arzneiformen besitzen feste Arzneiformen jedoch auch Nachteile. So entwickelt ein Teil der Patienten Widerwillen gegen das Schlucken von festen Darreichungsformen wie z. B. Tabletten, Kapseln und Dragees, so daß diese Patienten flüssige Arzneiformen vorziehen würden. Außerdem kann die Freisetzung des arzneilichen Wirkstoffs am Ort der Freisetzung wegen der dadurch bedingten hohen lokalen Konzentration die Gewebe reizen, insbesondere die Gewebe des Dünndarms. Dies ist selbst durch den Einsatz von sog. Pellets und Mikropellets nicht völlig vermeidbar.

Bei fettlöslichen Arzneimitteln kommt noch hinzu, daß ihre Resorption meist nur sehr unvollständig ist, von Patient zu Patient stark schwanken kann und sogar bei einem einzelnen Patienten, abhängig von seiner zusätzlichen Nahrungsaufnahme, von Tag zu Tag schwanken kann. Dies liegt daran, daß die Resorption von fettlöslichen (lipophilen) Arzneimitteln stark abhängig ist von der Gallenproduktion und der Nahrungsaufnahme der Patienten, so daß es zu unerwünschten hohen intra- und interindividuellen Blutspiegelschwankungen des Arzneimittels kommen kann.

Flüssige Arzneiformen sind bekannt zur oralen Anwendung wasserlöslicher Arzneimittel in vorwiegend wäßrigen Lösungen, gelegentlich in alkoholisch wäßrigen Lösungen. Fettlösliche Arzneimittel werden gelegentlich auch in flüssiger Form zur oralen Anwendung verabreicht, wobei das Arzneimittel meist in einem Öl natürlicher Herkunft gelöst wird und häufig noch zusätzlich Lösungsvermittler wie Ethanol und/oder Poly(oxyethylen)-Derivate von Ölen und Fetten, z. B. Poly(oxyethylen)-6-glycerol-tri(oleat, linolat) und/oder Polyoxyethylen-40-Rizinusöl verwendet werden. Solche Lösungen sind wegen ihres öligen Geschmacks und ihrer öligen Konsistenz unangenehm beim Einnehmen, die notwendigen Lösungsvermittler können unerwünschte Nebenwirkungen, z. B. allergische Reaktionen, auslösen und zudem bestehen auch hier die oben beschriebenen Probleme der gallen- und nahrungsabhängigen Resorption mit der Folge der hohen intra- und interindividuellen Blutspiegelschwankungen des Wirkstoffs. Außerdem könnten sie die Resorption von anderen Stoffen, z. B. Vitaminen, Paraffinen, beeinflussen.

Es ist daher von großer Bedeutung flüssige Darreichungsformen fettlöslicher (lipophiler) Arzneimittel zu Verfügung zu haben, die leicht einzunehmen sind, eine kontrollierte Freigabe des Wirkstoffs erlauben, wobei die Freigabe des Wirkstoffs in verzögerter (retardierter) Form sowohl teilweise im Magen und im Dünndarm sowie in kontrollierter Weise ausschließlich im Dünndarm erfolgt sowie außerdem eine bessere Verfügbarkeit des Wirkstoffs (bessere Resorption) besitzen und außerdem intra- und interindividuelle Schwankungen der Resorption vermeiden. Zudem sollte die Verwendung evtl. schädlicher Hilfsstoffe wie Ethanol und/oder Poly(oxyethylen)-Derivate vermieden werden.

Die beschriebenen Probleme werden überraschenderweise gelöst durch die hier vorliegende Erfindung, in der fettlösliche Arzneimittel enthaltende Zubereitungen zur oralen Applikation beschrieben werden, die ein oder mehrere fettlösliche (lipophile) Arzneimittel, ein oder mehrere Öle natürlicher Herkunft, Phosphatidylcholin und/oder Phosphatidylethanolamin und Wasser enthalten.

Eine Aufgabe der vorliegenden Erfindung ist es daher, eine fettlösliche (lipophile) Arzneimittel enthaltende flüssige Zubereitung zur oralen Applikation bereitzustellen, die eine bessere (höhere) und eine gleichmäßigere Resorption lipophiler Arzneimittel bei oraler Anwendung ermöglicht und zudem die bei lipophilen Arzneimitteln häufig auftretenden inter- und intraindividuellen Blutspiegelschwankungen des resorbierten Arzneimittels vermindert.

Eine weitere Aufgabe der vorliegenden Erfindung ist es, eine fettlösliche Arzneimittel enthaltende flüssige Zubereitung bereitzustellen, bei der das lipophile Arzneimittel nur teilweise im Magen, vorwiegend jedoch im Dünndarm freigesetzt wird und damit zumindest teilweise magensaftresistent ist und eine verzögerte (retardierte) Freisetzung besitzt.

Weitere Aufgaben der vorliegenden Erfindung sind, möglicherweise auftretende Reizungen des Magens und Darms durch das (die) Arzneimittel zu vermindern und/oder zu vermeiden, auf zusätzliche evtl. schädliche Hilfsstoffe wie z. B. Poly(oxyethylen)-6-glycerol-tri(oleat, linolat) und/oder Poly(oxyethylen)-40-Rizinusöl und/oder Ethanol verzichten zu können.

Diese Aufgabe(n) wird (werden) erfindungsgemäß dadurch gelöst, daß eine lipophile Arzneimittel enthaltende pharmazeutische Zubereitung zur Verfügung gestellt wird, in der ein oder mehrere Arzneimittel, ein oder mehrere Öle natürlicher Herkunft, 3-sn-Phosphatidylcholin und/oder Phosphatidylethanolamin und Wasser in einer Öl-in-Wasser-Emulsion enthalten sind, wobei sich die lipophilen Arzneimittel in der öligen Phase befinden.

Mit der erfindungsgemäßen Zubereitung zur oralen Applikation erhält man überraschenderweise eine bessere und zudem gleichmäßigere, d. h. exakter dosierbare Wirksamkeit des fettlöslichen Arzneimittels. Damit kann die Dosis vermindert werden und gleichzeitig die Schwere eventuell vorhandener Nebenwirkungen. Zudem kann, falls dies notwendig erscheint, auf die zusätzliche Verwendung evtl. schädlicher Hilfsstoffe wie z. B. Ethanol und/oder Poly(oxyethylen)-Derivate verzichtet werden.

Die höhere und gleichmäßigere Resorption der lipophilen Arzneimittel wird möglicherweise dadurch errreicht, daß die lipophilen Arzneimittel in der erfindungsgemäßen Zubereitung in der Mikrosphäre eines Fettpartikels gelöst sind und dieses Fettpartikel im Magen-Darm-Trakt in einer Weise aufgespalten bzw. resorbiert werden, wie dies auch mit natürlichen Nahrungsmitteln, z. B. Milch, die ebenfalls eine Öl-in-Wasser-Emulsion darstellt, geschieht.

Außerdem ist es mit der erfindungsgemäßen pharmazeutischen Zubereitung möglich, die Geschwindigkeit der Resorption zu steuern. Die Fettpartikel der Emulsion, in denen das (die) lipophile(n) Arzneimittel enthalten ist (sind), können nämlich durch Variation von Qualität und/oder Quantität des verwendeten 3-sn-Phosphatidylcholins so hergestellt werden, daß die Freisetzung des lipophilen Arzneimittels schnell oder langsam erfolgt. So ist es beispielsweise möglich, die pharmazeutische Zubereitung so zusammenzusetzen, daß die Fettpartikel bereits teilweise im Magen zerfallen und das lipophile Arzneimittel bereits teilweise im Magen freigesetzt wird, oder aber in einer solchen Weise, daß die Fettpartikel den Magen größtenteils unverändert passieren und die Freisetzung und Resorption erst im Dünndarm erfolgt. Damit erhält man im Prinzip eine magensaftresistente Form einer pharmazeutischen Zubereitung. Damit erweitern sich die Anwendungsmöglichkeiten beträchtlich, da man damit lipophile Arzneimittel auch erst im Darm freisetzen kann und dadurch eine den physiologischen Verdauungsvorgängen vergleichbare Darreichungsform erhält.

Die erfindungsgemäße pharmazeutische Zubereitung zur oralen Anwendung enthält therapeutische Mengen eines oder mehrerer lipophiler Arzneimittel, ein pharmazeutisch verträgliches Öl, 3-sn-Phosphatidylcholin, Wasser und gegebenenfalls freie Fettsäuren und/oder Alkalisalze freier Fettsäuren.

Gegebenenfalls können zusätzlich nichtionische Coemulgatoren und Lösungsmittel wie z. B. Poly(oxyethylen)-6-glycerol-tri(oleat, linolat) und/oder Poly(oxyethylen)-40-Rizinusöl zugesetzt werden.

Bei den erfindungsgemäßen pharmazeutischen Zubereitungen handelt es sich um Öl-in-Wasser-Emulsionen, bei denen die Tröpfchengröße der einzelnen Fettpartikel unter 20 Mikron, bevorzugt unter 5 Mikron und besonders bevorzugt im Mittel zwischen 0,05 und 2 Mikron liegt. Ganz besonders bevorzugt sind mittlere Größen der Fettpartikel im Durchmesser zwischen 0,10 und 1 Mikron.

Als Öle werden vorwiegend Öle natürlicher Herkunft verwendet wie z. B. Sojaöl und/oder Maiskeimöl und/oder Weizenkeimöl und/oder Distelöl und/oder Olivenöl und/oder Baumwollsamenöl und/oder Nußöl und/oder Fischöle und/oder MCT-Öle (mittelkettige Triglyceride = Kokosöl) und/oder hydrierte und/oder teilhydrierte Öle.

Als Lieferanten der Phosphatidylcholine können beispielweise pflanzliche Lecithine aus Soja und/oder Raps, Lecithin aus Ei und/oder Hirn dienen. Bevorzugt sind Sojalecithin und Eilecithin. Die verwendeten Phosphatidylcholine und Lecithine können auch teilhydriert oder hydriert sein.

Da lipidlösliche Arzneimittel in natürlichen Ölen löslich sind, liegt es nahe, diese einfach in solchen Ölen zu lösen und zu verabreichen. Dies bereitet jedoch Probleme, da viele Patienten das Schlucken von reinem Öl verweigern. Außerdem ist die Resorption relativ gering und teilweise abhängig von der Art der aufgenommenen Nahrung und der Gallensekretion des Patienten.

In der erfindungsgemäßen pharmazeutischen Zubereitung liegt das lipophile Arzneimittel dagegen in der öligen Phase einer Öl-in-Wasser-Emulsion vor. Eine natürlich vorkommende Öl-in-Wasser-Emulsion ist beispielsweise Milch. Bei Zugabe eines fettlöslichen (lipophilen) Arzneimittels zu Milch gelingt es jedoch nicht, ein solches Arzneimittel darin zu lösen, da selbst nach intensivem Rühren der weitaus überwiegende Teil noch ungelöst ist. Dies dürfte darauf zurückzuführen sein, daß das lipophile Arzneimittel die äußere Hülle der Fettpartikel nicht durchdringen kann, d. h. ein "Beladen" der Fettpartikel mit dem Arzneimittel nicht möglich ist.

Aus der EP-A-0 391 269 sind Öl-in-Wasser-Emulsionen bekannt, die unter anderem Cyclosporine enthalten können. Diese pharmazeutische Zubereitung ist dadurch gekennzeichnet, daß sie etwa 3-50 % eines Trägeröls enthält, bestehend aus einem MCT-Öl, wahlweise in Kombination mit einem Pflanzenöl, etwa 0,05-20 % eines Phospholipids, etwa 0,03-10 % eines nicht-ionischen Tensids und etwa 0,05-5 % eines ionischen Tensids. Die Verwendung ionischer und nicht-ionischer Tenside ist ein obligatorischer Bestandteil der pharmazeutischen Zusammensetzung der EP-A-0 391 269.

Derartige Tenside (Detergenzien) sind jedoch in der erfindunggemäßen pharmazeutischen Zubereitung unbedingt zu vermeiden. Aufgrund ihrer toxischen Wirkungen sind sie kontraindiziert! Dies gilt sowohl für orale als auch parenterale erfindungsgemäße Zubereitungen.

Die US 5 206 219 offenbart pharmazeutische Zubereitungen zur oralen Applikation, die ein hydrophiles oder ein hydrophobes Tensid enthalten. Derartige Tenside sind jedoch, wie oben ausgeführt, erfindungsgemäß kontraindiziert. Weiterhin enthält die pharmazeutische Zubereitung der US 5 206 219 Propylenglykol und/oder Ethylenglykol in einer Menge von 30 - 60 %. Diese sind intravenös wegen schwerer Nebenwirkungen absolut kontraindiziert. Außerdem enthält diese pharmazeutische Zubereitung keine natürlichen Öle, sondern freie Fettsäuren in Mengen von 30 - 60 %. Freie Fettsäuren, z.B. Ölsäure, können in derart hohen Mengen intravenös nicht verabreicht werden, da hier die Gefahr einer Hämolyse des Blutes besteht.

Erfindungsgemäß sind demnach oral oder parenteral ionische und nicht-ionische Detergenzien zu vermeiden. Weiterhin sind Polyole wie Propylenglykol und Polyethylenglykol zu vermeiden. Ein besonderer Vorteil der erfindungsgemäßen pharmazeutischen Zubereitung zur oralen Applikation liegt darin, daß bei Verwendung von Sojalecithin die Beimengung eines Alkalisalzes einer freien Fettsäure nicht notwendig ist.

Es wurde nun überraschenderweise erfindungsgemäß gefunden, daß es möglich ist, eine oral anwendbare Applikationsform in Form einer Emulsion von lipophilen Arzneimitteln in natürlich pharmazeutisch verträglichen Ölen, 3-sn-Phosphatidylcholin und Wasser herzustellen. Dabei wird (werden) das (die) verwendete(n) Arzneimittel zunächst in dem verwendeten Öl oder Ölgemisch gelöst, anschließend unter Verwendung von 3-sn-Phosphatidylcholin, bevorzugt in Form von Sojalecithin und/oder Eilecithin, emulgiert und das Gemisch in Wasser zu einer stabilen Emulsion mit einer Partikelgröße der Öl(Fett)-partikel von wengier als 20 Mikron, bevorzugt im Mittel zwischen 0,05 und 2 Mikron, insbesondere bevorzugt im Mittel zwischen 0,1 und 1 Mikron, homogenisiert.

Ist eine sterile oder weitgehend sterile Emulsion zur oralen Anwendung erwünscht, kann eine Sterilisation durch Hitze erfolgen, bzw. eine Sterilisation und/oder Konservierung durch entsprechende Filtration und/oder Verwendung eines Konservierungsmittels.

Die erhaltene pharmazeutische Zubereitung zur oralen Anwendung enthält keine bedenklichen Hilfsstoffe, ermöglicht eine bessere und/oder gleichmäßigere Resorption des (der) Arzneimittel, erlaubt eine gezielte, evtl. verzögerte Freisetzung und vermeidet weitgehend Schädigungen (z. B. Reizungen) der Gewebe des Magen-Darm-Traktes.

Jedes überwiegend fettlösliche (lipophile) Arzneimittel oder auch mehrere lipophile Arzneimittel können für die erfindungsgemäße Zubereitung bzw. das erfindungsgemäße Arzneimittel verwendet werden.

Beispiele für fettlösliche (lipophile) Arzneimittel können sein ohne auf diese Beispiele erfindungsgemäß beschränkt zu sein, Calciumantagonisten wie z. B. Nifedipin, Nitrendipin und Nimodipin, Antitumormittel wie z. B. Mitomycin, Bleomycin und Doxorubicin, Antibiotika wie z. B. Penicillin, Erythromycin, Streptomycin, Tetracyclin, Cycoserin, Immunsuppressiva wie z. B. Cyclosporin, Antirheumatika wie z. B. Indomethacin, Piroxicam, Diclofenac, Ibuprufen, Ketoprofen, Penylbutazon, Hormone wie z. B Prostaglandine und synthetische Steroide, Psychotherapeutika wie z. B. Diazepam, Vitamine (als Arzneimittel) und Vitaminderivate wie z. B. Calciferol, Calcitriol und Secalciferol. Entscheidend ist, daß das verwendete Arzneimittel einen lipophilen Charakter hat und damit überwiegend fettlöslich ist. Überwiegend wasserlösliche Arzneimittel können dagegen nicht verwendet werden.

Als Öle natürlicher Herkunft können beispielsweise verwendet werden Sojaöl, Distelöl (Safloröl), Kokosöle (MCT-Öle), Fischöle, Maiskeimöl, Weizenkeimöl, Olivenöl, Sonnenblumenöl, Baumwollsaatöl, Walnußöl, Leinsamenöl, Nachtkerzenöl und/oder Gemische dieser Öle. Die Öle können auch hydriert oder teilhydriert sein.

Die verwendeten Öle sollten arm an Peroxid und arm an Sauerstoff sein, um ein Ranzigwerden weitgehend zu vermeiden. Antioxidationsmittel wie z. B. Vitamin E und/oder andere Antioxidantien können enthalten sein.

Das (Die) verwendete(n) Arzneimittel wird (werden) zunächst in dem Öl, z. B. Sojaöl, gelöst, evtl. in der Wärme. Die verwendeten Konzentrationen des Arzneimittels hängen dabei von der Art des Arzneimittels bzw. der gewünschten therapeutischen Dosierung ab. Beim Lösungsvorgang sollte darauf geachtet werden, daß das Vorhandensein von Sauerstoff möglichst ausgeschlossen wird, um eine Oxidation des Öls zu vermeiden.

Zur Erzielung höherer Konzentrationen des verwendeten Arzneimittels können dem Öl zusätzlich noch Polyoxyethylen-Derivate, z. B. Polyoxyethylenglykole, Poly(oxyethylen)-6-glycerol-tri(oleat, linolat) und/oder Poly(oxyethylen)-40-Rizinusöl und/oder Sorbitan-Derivate (vgl. dazu Pharm. Martindale, 28th Edition, S. 376 ff) zugegeben werden. Zur Herstellung der erfindungsgemäßen pharmazeutischen Zubereitung sind solche Hilfsstoffe jedoch nicht unbedingt notwendig.

Zu der das Arzneimittel enthaltenden öligen Lösung wird nun Wasser, gegebenenfalls keinen Sauerstoff enthaltend, gegeben sowie als Emulgator 3-sn-Phosphatidylcholin, bevorzugt in Form von Sojalecithin und/oder Eilecithin. Auf 100 Teile Öl werden im allgemeinen 4 - 20 Teile des 3-sn-Phosphatidylcholins gegeben. Dieses kann auch hydriert oder teilhydriert sein.

Als Lieferanten des 3-sn-Phosphatidylcholins und/oder teilhydrierter oder hydrierter Choline können beispielsweise Lecithine aus Soja und/oder Raps und/oder Ei und/oder Hirn dienen. Bevorzugt sind Sojalecithin und/oder Eilecithin.

Besonders bevorzugt sind Eilecithin mit einem Gehalt an 3-sn-Phosphatidiylcholin von 50 - 85 % sowie Sojalecithin mit einem Gehalt an 3-sn-Phosphatidylcholin von 20 - 80 %.

Gegebenenfalls kann außerdem eine freie Fettsäure und/oder ein Alkalisalz einer freien Fettsäure mit 6 - 26 Kohlenstoffatomen zugegeben werden, um den pH-Wert auf ca. 4 - 10, bevorzugt 5,5 bis 9 einzustellen und die Emulgierung und Homogenisierung zu erleichtern. Bevorzugt sind als freie Fettsäuren und/oder deren Alkalisalze, insbesondere Kalium und/oder Natriumsalze, die Palmitinsäure, Palmitoleinsäure, Stearinsäure, Ölsäure, Linolsäure und/oder Linolensäure. Auch Gemische dieser Fettsäuren können eingesetzt werden.

Das Gemisch, welches nun (ein) fettlösliche(s) Arzneimittel, Öl(e) natürlicher Herkunft, 3-sn-Phosphatidylcholin, Wasser und gegebenenfalls freie Fettsäuren und/oder deren Alkalisalze, wird nun mit Wasser soweit verdünnt, daß das Öl 5 - 80 % des Gesamtgewichts, bevorzugt 20 - 60 % des Gesamtgewichts beträgt.

Es wird nun durch starkes Rühren, bevorzugt mit einem Ultra-Turrax, eine Rohemulsion hergestellt. Diese Rohemulsion wird nun mit einem Hochdruck-Homogenisator bei Drücken zwischen 100 - 500 bar so lange, unter Umständen mehrmals, homogenisiert, bis man eine Emulsion erhält, bei der die Partikelgröße aller Partikel unter 20 Mikron, bevorzugt unter 10 Mikron, besonders bevorzugt unter 5 Mikron liegt. Dabei haben die Fettpartikel im Mittel eine Größe von 0,05 - 10 Mikron, bevorzugt zwischen 0,0,5 - 2 Mikron besonders bevorzugt zwischen 0,1 und 1 Mikron. Der verwendete Hochdruck-Homogenisator sollte vorzugsweise drei Kolben besitzen.

Danach wird die Emulsion gegebenenfalls durch Wasser auf die gewünschte Konzentration verdünnt. Anschließend kann die Emulsion in geeignete Darreichungsformen, z. B. in Trinkfläschchen oder Trinkampullen abgefüllt werden. Auch eine Abfüllung der Emulsion in Weichgelatine-Kapseln ist beispielsweise möglich.

Um die orale Darreichungsform vor der Zersetzung durch Bakterien zu schützen, kann gegebenenfalls eine Hitzesterilisation oder -konservierung durchgeführt werden und/oder die pharmazeutische Zubereitung wird durch den Zusatz üblicher Konservierungsmittel wie z. B. Benzoesäure, Sorbinsäure, p-Hydroxybenzoesäureester usw. geschützt.

Um den Geschmack zu verbessern, kann die Emulsion durch die Zugabe von Aromastoffen gegebenenfalls aromatisiert werden. Außerdem können zur Geschmacksverbesserung z. B. Zucker wie z. B. Saccharose, Glukose, Sorbit, Xylit und/oder künstliche Süßstoffe wie z. B. Saccharin und/oder auch mehrwertige Alkohole wie z. B. Glycerin zugegeben werden.

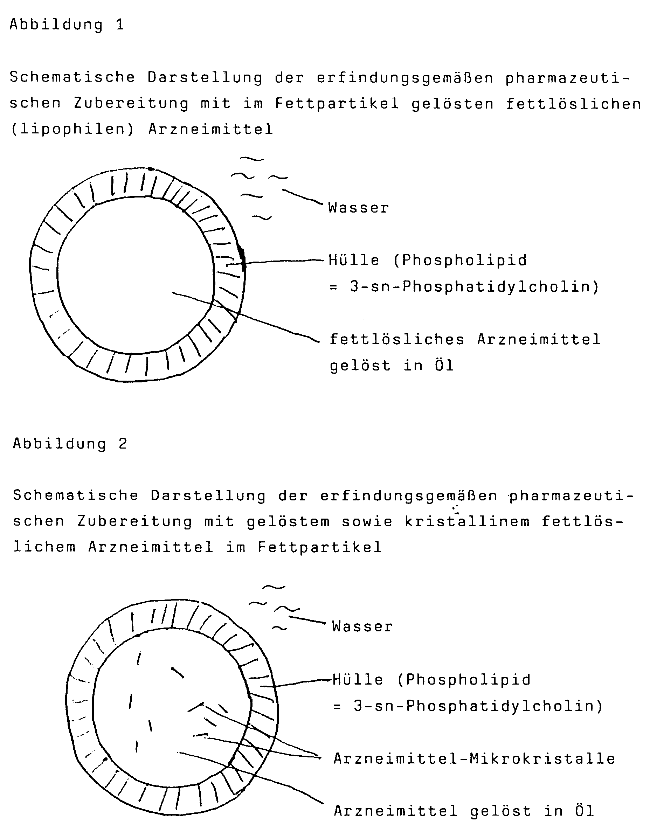

Üblicherweise und vorzugsweise liegt dies in der pharmazeutischen Zubereitung enthaltene fettlösliche Arzneimittel in der öligen Phase gelöst vor. Dabei ist das Arzneimittel, gelöst im Öl, im Innern eines Fettpartikels enthalten, wobei das Öl mit einer Hülle, bestehend im wesentlichen aus Phospholipid (3-sn-Phosphatidylcholin), umgeben ist. Die beiliegende Abbildung 1 zeigt in schematischer Form den Aufbau der erfindungsgemäßen, das (die) fettlösliche(n) Arzneimittel enthaltenden Fett(Öl)-Partikel.

Es ist jedoch sogar auch möglich, das (die) fettlösliche(n) Arzneimittel bei der Herstellung in so hoher Konzentration anzuwenden, daß in der schließlich erhaltenen pharmazeutischen Zubereitung zur oralen Anwendung nur ein Teil des (der) Arzneimittel(s) gelöst vorliegt, ein anderer Teil sich jedoch ungelöst, bevorzugt in fester Form, im Inneren des Fettpartikels befindet. Dies wird in der Weise erreicht, daß bei erhöhter Temperatur (z. B. 50 - 100°C) das (die) Arzneimittel im Öl gelöst wird (werden) und damit gegebenenfalls eine bei Raumtemperatur dann übersättigte Lösung erhalten wird. Anschließend wird bei erhöhter Temperatur emulgiert und homogenisiert. Nach Herstellung der Emulsion erfolgt eine Abkühlung auf Raumtemperatur, wobei ein Teil des sich jetzt im Innern des Fettpartikels enthaltenen Arzneimittels auskristallisiert. Wegen der das Fettpartikel umgebenden Hülle, welche das nun in fester (oder flüssiger) Form vorliegende Arzneimittel nicht durchdringen kann, bleibt das Arzneimittel eingeschlossen. Die beiliegende Abbildung 2 zeigt in fester Form ein solches Fettpartikel.

Die pharmazeutische Zubereitung zur oralen Anwendung kann wie üblich verabreicht werden, z. B in Form von Trinkampullen, Tropfen zur oralen Anwendung usw. Auch eine Abfüllung in Weichgelatine-Kapseln ist möglich. Im Magen-Darm-Trakt wird das im Fettpartikel enthaltene Arzneimittel freigesetzt und resorbiert.

Die folgenden Beispiele beschreiben die erfindungsgemäße pharmazeutische Zubereitung und das Verfahren zu ihrer Herstellung im einzelnen. Die Erfindung ist darauf nicht beschränkt.

### Beispiel 1

10,0 kg winterisiertes Sojaöl (neutraler pH-Wert, peroxidfrei) wird mit Stickstoff begast und unter Rühren und weiterem Begasen mit Stickstoff auf ca. 50°C erwärmt. Unter winterisiertem Sojaöl wird Sojaöl verstanden, das vor der Anwendung auf unter -10°C abgekühlt wird, damit in der Kälte unlösliche Anteile ausfallen, die abfiltriert werden.

Zu diesem Sojaöl werden 20 g Nifedipin gegeben und unter Rühren gelöst.

In ein zweites Gefäß werden 12,5 kg Wasser gegeben. Es wird mit Stickstoff begast, um den Sauerstoffgehalt des Wassers zu verringern. Es werden nun 1,2 kg Eilecithin (Jodzahl ca. 60 - 70) mit einem Gehalt von ca. 75 % 3-sn-Phosphatidylcholin und ca. 15 % Phosphatidylethanolamin (Kephalin) zugegeben und 50 g Natriumoleat. Durch starkes Rühren mit einem Ultra-Turrax wird bei einer Temperatur von ca. 50°C eine Rohemulsion hergestellt.

Nun wird das Sojaöl mit dem darin gelösten Nifedipin durch ein fettresistentes Filter mit einer Porengröße von ca. 50 Mikron in dieses zweite Gefäß gegeben und weitere 10 Minuten bei ca. 50°C mit einem Ultra-Turrax stark gerührt. Der pH-Wert sollte nun zwischen 5 - 10 liegen, bevorzugt zwischen 6 - 9. Ist dies nicht der Fall, werden gegebenenfalls noch etwas Natriumoleat bzw. Ölsäure zugegeben.

Die jetzt vorhandene Rohemulsion enthält Fettpartikel, deren Größe bis zu 20 Mikron beträgt, vorwiegend Fettpartikel von einer Größe zwischen 2 und 10 Mikron. Eine solche Emulsion ist gegebenenfalls erfindungsgemäß verwendbar. Durch weitere Homogenisierung, wie im folgenden beschrieben, kann die Partikelgröße verkleinert werden und damit die Resorption und sonstige Eigenschaften der erfindungsgemäßen pharmazeutischen Zubereitung weiter verbessert werden.

Die erhaltene oben beschriebene Emulsion wird durch ein Edelstahlfilter (Porengröße 5 - 50 Mikron) filtriert und anschließend mit einem 2-Stufen-Hochdruck-Homogenisator mit drei Kolben homogenisiert bei Drücken zwischen 100 und 500 bar. Bereits bei der ersten Homogenisierung ist die Partikelgröße fast aller Fettpartikel unter 1,5 Mikron, einzelne Partikel besitzen jedoch noch Durchmesser bis zu 10 Mikron.

Der Vorgang der Homogenisierung wird so oft wiederholt, bis die gewünschte Partikelgröße erreicht wird. Nach 3-4maligem Homogenisieren ist kein Partikel mehr größer als 5 Mikron, im Mittel beträgt der Durchmesser der Fettpartikel 0,1 - 0,6 Mikron.

Es werden nun noch 27,5 kg stickstoffgesättigtes Wasser zugegeben und durch ein Edelstahlfilter mit einer Porengröße von 5 bis 10 Mikron filtriert. Man erhält so 50 l der erfindungsgemäßen pharmazeutischen Zubereitung, enthalten 20 g Nifedipin.

Die Partikelgröße und die Partikelverteilung der erfindungsgemäßen pharmazeutischen Zubereitung beträgt nach 3-4maligem Homogenisieren in etwa:

| Partikelgröße | Zahl der Teilchen |
|---|---|
| < 0,2 Mikron | 30 % |
| 0,2 - 0,5 Mikron | 47 % |
| 0,5 - 0,9 Mikron | 14 % |
| 0,9 - 1,2 Mikron | 6 % |
| 1,2 - 1,9 Mikron | 2 % |
| 1,9 - 2,2 Mikron | unter 1 % |
| 2,2 - 2,5 Mikron | unter 1 % |
| 2,5 - 3,2 Mikron | unter 1 % |
| > 3,2 Mikron | unter 1 % |

Die erhaltene erfindungsgemäße pharmazeutische Zubereitung kann zusammen mit einem Konservierungsmittel beispielsweise in 100-ml-Fläschchen oder Trinkampullen abgefüllt werden, gegebenenfalls ist auch eine Hitzesterilisation möglich, wobei die Hitze die Partikelverteilung nicht wesentlich verändert. Die erhaltene erfindungsgemäße pharmazeutische Zubereitung enthält 40 mg Nifedipin pro 100 ml.

### Beispiel 2

Das Verfahren des Beispiels 1 wird wiederholt, wobei statt des Sojaöls ein Fischöl-Konzentrat, enthaltend 18 % Eicosapentaensäure und 12 % Docosahexaensäure, verwendet wird. Man erhält eine entsprechende pharmazeutische Zubereitung.

### Beispiel 3

Das Verfahren des Beispiels 1 wird wiederholt, wobei statt des Nifedipins 15 g Nimodipin verwendet werden.

### Beispiel 4

Das Beispiel 1 wird wiederholt, wobei statt des Eilecithins Sojalecithin mit einem Gehalt von ca. 60 % 3-sn-Phosphatidylcholin eingesetzt wird.

### Beispiel 5

Das Beispiel 1 wird wiederholt, wobei statt Nifedipin 30 g Cyclosporin verwendet werden.

### Beispiel 6

10 kg winterisiertes Sojaöl mit einem Gehalt an freien Fettsäuren von 2,5 meq/l und einem pH-Wert von ca. 3,5 wird auf ca. 50°C erwärmt und es werden 20 g Nifedipin zugegeben. Entsprechend Beispiel 1 wird eine Rohemulsion mit einem Ultra-Turrax hergestellt. Anschließend wird durch Zugabe einer 10%igen Natriumhydroxidlösung ein pH-Wert von 6,5 - 8,5 hergestellt. Die weitere Herstellung erfolgt nach Beispiel 1.

Bei der parenteralen Applikation wird die nachfolgende pharmazeutische Zusammensetzung bevorzugt eingesetzt:

| | pro Liter |
|---|---|
| 1. Sojaöl | bis 400 g |
| 2. Eilecithin | 5- 20 g |
| dabei muß das Eilecithin zwischen 60 % und 85 % Phosphatidylcholin enthalten | |
| 3. Natriumsalz oder Kaliumsalz einer Fettsäure mit mindestens 10 Kohlenstoffatomen: 0,2 - 1,0 g. Statt des Salzes kann auch eine Alkalilauge + Fettsäure verwendet werden. | |

In der erfindungsgemäßen pharmazeutischen Zubereitung zur oralen Applikation liegt das teilweise hydrierte 3-sn-Phosphatidylcholin bevorzugt in der Form von teilweise hydriertem Sojalecithin oder teilweise hydriertem Eilecithin vor, wobei vorzugsweise das Eilecithin mindestens 60 Gew.% 3-sn-Phosphatidylcholin, weiterhin bevorzugt zwischen 60 - 85 Gew.% enthält.

Der Gehalt an 3-sn-Phosphatidylcholinen in der erfindungsgemäßen pharmazeutischen Zubereitung liegt bevorzugt bei 0,3 - 4, weiterhin bevorzugt bei 0,6 - 2,4 Gew.%. Der Gehalt der 3-sn-Phosphatidylcholin enthaltenden Substanzen in der erfindungsgemäßen pharmazeutischen Zubereitung liegt bevorzugt bei 0,5 - 6,5, weiterhin bevorzugt bei 1 - 4 Gew.%.

## Patentansprüche

1. Fettlösliche (lipophile) Arzneimittel enthaltende pharmazeutische Zubereitung zur oralen Applikation,
dadurch gekennzeichnet,
daß sie ein oder mehrere fettlösliche (lipophile) Arzneimittel, ein oder mehrere Öle natürlicher Herkunft, 3-sn-Phosphatidylcholin und/oder Phosphatidylethanolamin und Wasser enthält.

2. Fettlösliche (lipophile) Arzneimittel enthaltende pharmazeutische Zubereitung zur oralen Applikation nach Anspruch 1,
dadurch gekennzeichnet,
daß zusätzlich pharmazeutisch verträgliche freie Fettsäuren und/oder Alkalisalze freier Fettsäuren enthalten sind.

3. Fettlösliche (lipophile) Arzneimittel enthaltende pharmazeutische Zubereitung zur oralen Applikation nach den Ansprüchen 1 und/oder 2,
dadurch gekennzeichnet,
daß das 3-sn-Phosphatidylcholin in Form von 3-sn-Phosphatidylcholin enthaltenden Substanzen vorliegt, und daß vorzugsweise die 3-sn-Phosphatidylcholin enthaltende Substanz Sojalecithin (Sojaphosphatid) und/oder Eilecithin ist.

4. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß das verwendete 3-sn-Phosphatidylcholin teilweise oder vollständig hydriert ist.

5. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß als Öle natürlicher Herkunft Sojaöl und/oder Maiskeimöl und/oder Weizenkeimöl und/oder Distelöl und/oder Olivenöl und/oder Sonnenblumenöl und/oder Nußöl und/oder Fischöl und/oder deren teilhydrierten oder hydrierten Öle und/oder mittelkettige Triglyceride verwendet werden und daß vorzugsweise das Öl frei von Peroxiden ist.

6. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß das (die) Arzneimittel in einer therapeutisch wirksamen Konzentration vorliegt(en).

7. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß das natürliche Öl in einem Anteil von 5 - 80 %, bevorzugt in einem Anteil von 20 - 60 % des Gesamtgewichts, in der pharmazeutischen Zubereitung vorliegt.

8. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß als pharmazeutisch verträgliche Fettsäuren gesättigte und/oder ungesättigte Fettsäuren und/oder deren Alkalisalze, bevorzugt mit 6 - 26 Kohlenstoffatomen, enthalten sind.

9. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß der Gehalt an freien Fettsäuren und/oder deren Alkalisalzen zwischen 0,1 Gew.% und 3 Gew.%, bevorzugt zwischen 0,5 Gew.% und 1,5 Gew.%, beträgt.

10. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß die in den natürlichen Ölen und/oder hydrierten und/oder teilhydrierten natürlichen Ölen enthaltenen Arzneimittel im Innern der aus diesen Ölen bestehenden Fettpartikel enthalten sind, von einer Hülle aus 3-sn-Phosphatidylcholin und/oder Phosphatidylethanol umgeben und in Wasser emulgiert sind.

11. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß die mittlere Größe der Fettpartikel in der Emulsion zwischen 0,05 und 10 Mikron, bevorzugt zwischen 0,05 und 2 Mikron und insbesondere bevorzugt zwischen 0,1 und 1 Mikron beträgt.

12. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß nach der oralen Verabreichung der pharmazeutischen Zubereitung ein wesentlicher Anteil der (des) Arzneimittel(s) nicht im Magen, sondern erst im Dünndarm freigesetzt wird.

13. Fettlösliche (lipophile) Arzneimittel enthaltende pharmazeutische Zubereitung zur oralen Applikation nach einem oder mehreren der vorhergehenden Ansprüche,
dadurch gekennzeichnet,
daß die fettlöslichen (lipophilen) Arzneimittel aus der Gruppe der Calciumantagonisten und/oder nichtsteroidalen Antirheumatika und/oder Antiphlogistika und/oder der Antibiotika und/oder ACE-Hemmer und/oder der Immunsuppressiva und/oder der Tumorhemmer und/oder der Prostaglandine und/oder der Hormone und/oder der synthetischen Steroide und/oder der Vitamin-Derivate stammen.

14. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß die fettlöslichen Arzneimittel in natürlichen Ölen gelöst und anschließend mit 3-sn-Phosphatidylcholin und/oder Phosphatidylethanolamin und Wasser in an sich bekannter Weise emulgiert werden.

15. Verfahren nach Anspruch 14,
dadurch gekennzeichnet,
daß die fettlöslichen Arzneimittel vor der Emulgierung in dem Öl natürlicher Herkunft vollständig gelöst und anschließend mit Hilfe von 3-sn-Phosphatidylcholinen und/oder deren Alkalisalzen in Wasser zu einer Rohemulsion emulgiert werden und das Gemisch anschließend zu einer Emulsion, bevorzugt mit einer Partikelgröße von unter 10 Mikron, insbesondere bevorzugt unter 2 Mikron homogenisiert wird.

16. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß das Gemisch aus Cyclosporin(en), Ölen natürlicher Herkunft, 3-sn-Phosphatidylcholin und Wasser bereits 0,01 bis 1 % an freien natürlichen Fettsäuren und/oder deren Alkelisalzen enthält und dieses Gemisch mittels Alkalilauge(n) vor der Emulgierung auf einen pH-Wert von 5 - 10, bevorzugt 6 - 9, eingestellt wird.

17. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß Fettsäuren und/oder, deren Alkalisalze verwendet werden, um die Emulsion auf einen pH-Wert von 5 - 10, bevorzugt 6 - 9, einzustellen.

18. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß zur Herstellung der pharmazeutischen Zubereitung im wesentlichen sauerstofffreie Lösungen verwendet werden.

19. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß zusätzlich pharmazeutisch verträgliche gesättigte und/oder ungesättigte Fettsäuren und/oder Alkalisalze der Fettsäuren verwendet werden.

20. Verfahren zur Herstellung der fettlösliche (lipophile) Arzneimittel enthaltenden pharmazeutischen Zubereitung zur oralen Applikation nach einem oder mehreren der vorhergehenden Ansprüche,
dadurch gekennzeichnet,
daß durch intensives Rühren zunächst eine Rohemulsion hergestellt wird und anschließend die endgültige Emulgierung in einem Hochdruck-Homogenisator bei Drücken zwischen 100 - 500 bar erfolgt, wobei vorzugsweise der Hochdruck-Homogenisator drei Kolben besitzt,

21. Verwendung der pharmazeutischen Zubereitung nach einem oder mehreren der vorhergehenden Ansprüche zur oralen Applikation.
